# EUROPEAN PATENT APPLICATION

(11) **EP 4 092 500 A1**
(43) Date of publication of application: **23.11.2022**
(21) Application number: 21761145.8
(22) Date of filing: 12.01.2021
(51) Int. Cl.: G05B 19/418

(54) **WEARABLE DEVICE CONTROL METHOD AND APPARATUS, ELECTRONIC DEVICE, AND READABLE STORAGE MEDIUM**

(30) Priority: 27.02.2020 CN 202010122804
(71) Applicant: GUANGDONG OPPO MOBILE TELECOMMUNICATIONS CORP., LTD., Dongguan, Guangdong 523860 (CN)
(72) Inventor: PU, Yuxiao, Dongguan, Guangdong 523860 (CN)
(74) Representative: Taor, Simon Edward William
(86) International application number: PCT/CN2021/071155
(87) International publication number: WO 2021/169636

(57) **Abstract**

Provided are a control and apparatus for a wearable device, an electronic device, and a computer-readable storage medium. The wearable device has a first operation mode and a second operation mode. The first operation mode is a mode for running a first system and a second system. The second operation mode is a mode for running only the second system. The first operation mode has a higher power consumption than the second operation mode. The control method includes: obtaining user behavior data (102), determining a user behavior status based on the user behavior data (104), and switching, in response to detecting that the user behavior status is a sleep status, the wearable device from the first operation mode to the second operation mode (106).

## Description

### CROSS-REFERENCES TO RELATED APPLICATIONS

This application claims priority to Chinese Patent Application No. 202010122804.4, filed with China National Intellectual Property Administration on February 27, 2020, titled "CONTROL METHOD FOR WEARABLE DEVICE, CONTROL APPARATUS FOR WEARABLE DEVICE, ELECTRONIC DEVICE AND COMPUTER-READABLE STORAGE MEDIUM", the entire disclosure of which is incorporated herein by reference.

### FIELD

The present disclosure relates to the field of computer technologies, and in particular, to a control method for a wearable device, a control apparatus for a wearable device, an electronic device, and a computer-readable storage medium.

### BACKGROUND

Smart wearable devices are becoming increasingly popular. Especially, smart watches and bracelets have been loved by more and more young people. The smart wearable devices not only have the functions of traditional watches, such as clocks, but also have some functions of other electronic devices, such as games and call functions of mobile phones. Thus, the smart wearable devices currently available on the market require high power consumption.

### SUMMARY

Embodiments of the present disclosure provide a control method for a wearable device, a control apparatus for a wearable device, an electronic device, and a computer-readable storage medium.

The present disclosure provides a control method for a wearable device. The wearable device has a first operation mode and a second operation mode. The first operation mode is a mode for running a first system and a second system. The second operation mode is a mode for running only the second system. The first operation mode has a higher power consumption than the second operation mode. The control method includes: obtaining user behavior data; determining a user behavior status based on the user behavior data; and switching, in response to detecting that the user behavior status is a sleep status, the wearable device from the first operation mode to the second operation mode.

The present disclosure further provides a control apparatus for a wearable device. The wearable device has a first operation mode and a second operation mode. The first operation mode is a mode for running a first system and a second system. The second operation mode is a mode for running only the second system. The first operation mode has a higher power consumption than the second operation mode. The control apparatus includes: an obtaining module configured to obtain user behavior data; a determining module configured to determine a user behavior status based on the user behavior data; and a switching module configured to switch, in response to detecting that the user behavior status is a sleep status, the wearable device from the first operation mode to the second operation mode.

The present disclosure further provides an electronic device. The electronic device includes a memory having a computer program stored thereon, and a processor. The processor is configured to, when executing the computer program, implement the following steps: obtaining user behavior data; determining a user behavior status based on the user behavior data; and switching, in response to detecting that the user behavior status is a sleep status, the wearable device from the first operation mode to the second operation mode.

The present disclosure further provides a computer-readable storage medium, having a computer program stored thereon. The computer program, when being executed by a processor, implements the following steps: obtaining user behavior data; determining a user behavior status based on the user behavior data; and switching, in response to detecting that the user behavior status is a sleep status, the wearable device from the first operation mode to the second operation mode.

With the above-mentioned control method and apparatus for the wearable device, the above-mentioned electronic device and the above-mentioned computer-readable storage medium, the user behavior data is obtained, and the user behavior status is determined based on the user behavior data. When it is detected that the user behavior status is the sleep status, the wearable device is switched from the second operation mode to the first operation mode. That is, in the sleep status, in order to switch the operation mode with higher power consumption to the operation mode with lower power consumption, the user does not need to manually switch, which saves the cost of user operation and lowers the power consumption of the wearable device.

### BRIEF DESCRIPTION OF DRAWINGS

In order to more clearly explain the embodiments of the present disclosure or the technical solutions in the related art, drawings used in the description of the embodiments or the related art are briefly described below. Obviously, the drawings as described below are merely some embodiments of the present disclosure. Based on these drawings, other drawings can be obtained by those skilled in the art without creative labor.
FIG. 1 is a flowchart of a control method for a wearable device in an embodiment.
FIG. 2 is a system architecture diagram of a wearable device in an embodiment.
FIG. 3 is a schematic diagram of an internal structure of a wearable device in an embodiment.
FIG. 4 is a schematic diagram illustrating system switching in an embodiment.
FIG. 5 is a schematic flowchart of a control method for a wearable device in another embodiment.
FIG. 6 is a structural block diagram of a control apparatus for a wearable device in an embodiment.
FIG. 7 is a schematic diagram of an internal structure of a wearable device in an embodiment.

### DESCRIPTION OF EMBODIMENTS

In order to clearly explain the purposes, technical solutions and advantages of the present disclosure, the present disclosure will be described in further detail below with reference to the accompanying drawings and embodiments. It should be understood that the specific embodiments described herein are only used to explain the present disclosure, but not to limit the present disclosure.

It should be understood that the terms "first", "second", etc. used in the present disclosure may be used herein to describe various elements, rather than limiting these elements. These terms are only used to distinguish a first element from another element. For example, without departing from the scope of the present disclosure, the first operation mode may be referred to as the second operation mode, and similarly, the second operation mode may be referred to as the first operation mode. Both the first operation mode and the second operation mode are operation modes, but they are not the same operation mode.

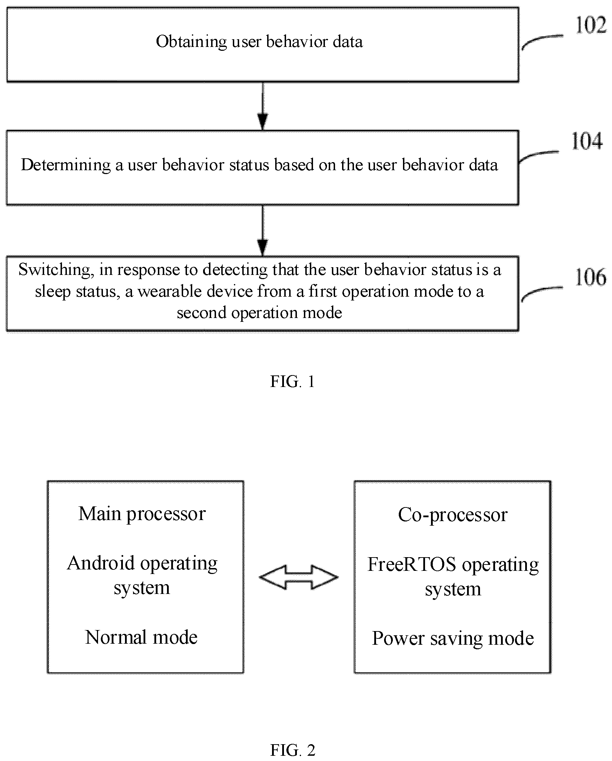

FIG. 1 is a flowchart of a control method for a wearable device in an embodiment. As an example, the control method for the wearable device in this embodiment is described in such a manner that the control method operates on the wearable device.

The wearable device has a first operation mode and a second operation mode. A power consumption of the first operation mode is higher than that of the second operation mode. The first operation mode is a mode for simultaneously running a first system and a second system. The second operation mode is a mode for running only the second system. Therefore, the first operation mode requires a higher power consumption than the second operation mode. Also, the first system has a higher power consumption than the second system. The first system is configured to implement functions that consume a lot of power, such as communication. The second system is configured to implement basic functions, such as step counting and heart rate detection. FIG. 2 is a system architecture diagram of a wearable device in an embodiment. For example, the first operation mode may be a normal mode, which supports functions such as communication; and the second operation mode may be a power saving mode, which supports functions such as step counting and heart rate detection. The first operation mode may also be referred to as a watch mode, and the second operation mode is referred to as a wristband mode. The system may be, but not limited to, an Android system, a Linux system, a Windows system, a WearOS system, an IOS system, a Real Time Operating System (RTOS). The first system has a higher power consumption than the second system. For example, the first system may be an Android operating system, and the corresponding second system may be an Embedded Real Time Operating System (FreeRTOS). In this case, the first operation mode is a mode, in which the wearable device runs the Android system and the RTOS system simultaneously; and the second operation mode is a mode, in which the wearable device switches off the Android system and only runs the RTOS system.

As illustrated in FIG. 1, the control method for the wearable device includes operations 102 to 106.

In operation 102, user behavior data is obtained.

The user behavior data refers to the data generated when the user performs a behavior. For example, the user behavior data may be, but not limited to, at least one of a heart rate value, an acceleration value, gyroscope data, and cardiopulmonary data. The acceleration value and the gyroscope data may be used to represent changes in the user's movements.

For example, in the first operation mode, the wearable device may obtain the user behavior data from the second system.

In operation 104, a user behavior status is determined based on the user behavior data.

The user behavior status may include a sleep status and an awake status. The awake status may refer to any status other than the sleep status, for example, but not limited to, the motion status.

For example, the wearable device may compare the user behavior data with a preset behavior data threshold to determine the user behavior status.

Alternatively, the user behavior data includes a user electrocardiosignal. The wearable device may extract a heart rate signal and a respiration signal from the user electrocardiosignal, and couples the heart rate signal with the respiration signal to obtain cardiopulmonary information. The user behavior status is determined based on the cardiopulmonary information, i.e., based on the continuous electrocardiosignal. The Fourier transform technique is adopted to analyze two characteristics of the signal: (1) a heart rate variability; (2) an amplitude fluctuation of electrocardiogram R wave (EDR) caused by respiration. By calculating the cross-spectral power and coherence of the two signals, the cardiopulmonary coupling dynamics spectrogram during sleep may be generated to evaluate the sleep quality and thus to determine the user behavior status. The wearable device may also obtain the acceleration value, to determine the user behavior status based on the cardiopulmonary information and the acceleration value. For example, when it is determined based on the cardiopulmonary information that the user behavior status is the sleep status, and when the acceleration value satisfies an acceleration threshold, it is determined that the user behavior status is the sleep status.

In operation 106, in response to detecting that the user behavior status is the sleep status, the wearable device is switched from the first operation mode to the second operation mode.

For example, when the wearable device detects that the user behavior status is the sleep status, the wearable device is automatically switched from the first operation mode to the second operation mode.

With the control method for the above wearable device, the user behavior data is obtained, and the user behavior status is determined based on the user behavior data. When it is detected that the user behavior status is the sleep status, the wearable device is switched from the second operation mode to the first operation mode. That is, in the sleep status, in order to switch the operation mode with higher power consumption to the operation mode with lower power consumption, the user does not need to manually switch, which saves the cost of user operation and lowers the power consumption of the wearable device.

In an embodiment, the user behavior data includes an acceleration value. Said determining the user behavior status based on the user behavior data includes: determining an average acceleration value within a first preset time period; and determining, in response to the average acceleration value being less than an acceleration threshold value, that the user behavior status is the sleep status.

The acceleration value may be measured by an acceleration sensor, for example, a high-precision accelerometer. The first preset time period may specifically refer to, but not limited to, 1 minute, 2 minutes, 5 minutes, etc. The first preset time period may be set as required. The first preset time period refers to a time period corresponding to the acceleration value. The average acceleration value within the first preset time period may be obtained by averaging the acceleration values within the first preset time period prior to the current moment. The acceleration threshold value may be an acceleration value when the user is in the sleep status. The acceleration value when the user is in the awake status is generally greater than the acceleration value when the user is in the sleep status. Thus, the user behavior status can be determined based on the acceleration value.

For example, the wearable device may obtain the acceleration value in real time and may determine the average acceleration value within the first preset time period. When the average acceleration value is less than the acceleration threshold value, it may be determined that the user behavior status is the sleep status. For example, the wearable device may determine the average acceleration value within 2 minutes, and when the average acceleration value within 2 minutes is less than the acceleration threshold value, it may be determined that the user behavior status is the sleep status.

With the control method for the wearable device in this embodiment, the average acceleration value within the first preset time period is determined, and when the average acceleration value is less than the acceleration threshold value, it is determined that the user behavior status is the sleep status. By means of the average acceleration value, the accidental situation of a single acceleration value may be avoided. For example, if the user turns over in sleep or scratches an itch, an instantaneous acceleration value may be high. However, by averaging the values, the accidental situations can be avoided, improving the accuracy of mode switching.

In an embodiment, the user behavior data includes a heart rate value. Said determining the user behavior status based on the user behavior data includes: determining an average heart rate within a second preset time period; determining, in response to the average heart rate being less than a heart rate threshold value, that the user behavior status is the sleep status.

The second preset time period may specifically refer to, but not limited to, 1 minute, 2 minutes, 5 minutes, etc. The second preset time period may be set as required. The second preset time period refers to a time period corresponding to the heart rate value. A duration of the second preset time period may be the same as that of the first preset time period. The average heart rate within the second preset time period may be obtained by averaging the heart rate values within the second preset time period prior to the current moment. The heart rate threshold value may be the heart rate value when the user is in a sleep status. The heart rate value when the user is in the awake status is generally greater than the heart rate value when the user is in the sleep status. Thus, the user behavior status can be determined based on the heart rate value.

For example, the wearable device may obtain the heart rate value in real time and may determine the average heart rate value within the second preset time period. When the average heart rate value is less than the heart rate threshold value, it may be determined that the user behavior status is the sleep status. For example, the wearable device may determine the average heart rate within 2 minutes, and when the average heart rate within 2 minutes is less than the heart rate threshold value, it may determine that the user behavior status is the sleep status.

With the control method for the wearable device in this embodiment, the average heart rate within the first preset time period is determined, and when the average heart rate is less than the heart rate threshold value, it is determined that the user behavior status is the sleep status. By means of the average heart rate, the accidental situation of a single heart rate value may be avoided, thereby improving the accuracy of mode switching.

In an embodiment, the user behavior data includes an acceleration value and a heart rate value. Said determining the user behavior status based on the user behavior data includes: determining an average acceleration value within the first preset time period and an average heart rate value within the second preset time period; and determining, in response to the average acceleration value being less than an acceleration threshold value and the average heart rate value being less than a heart rate threshold value, that the user behavior status is the sleep status.

The duration of the first preset time period may be the same as that of the second preset time period.

With the control method for the wearable device in this embodiment, the average acceleration value within the first preset time period and the average heart rate value within the second preset time period are determined. When the average acceleration value is less than the acceleration threshold value and the average heart rate value is less than the heart rate threshold value, it can be determined that the user behavior state is the sleep state. By determining the user behavior state being the sleep state when two conditions are satisfied at the same time, the accuracy of mode switching can be improved.

In an embodiment, the wearable device includes a first processor for running the first system, and a second processor for running the second processor. Said switching, in response to detecting that the user behavior status is the sleep status, the wearable device from the first operation mode to the second operation mode includes: controlling, in response to detecting that the user behavior status is the sleep status, a switch in the wearable device to disconnect the first processor from the second processor to switch the wearable device from the first operation mode to the second operation mode.

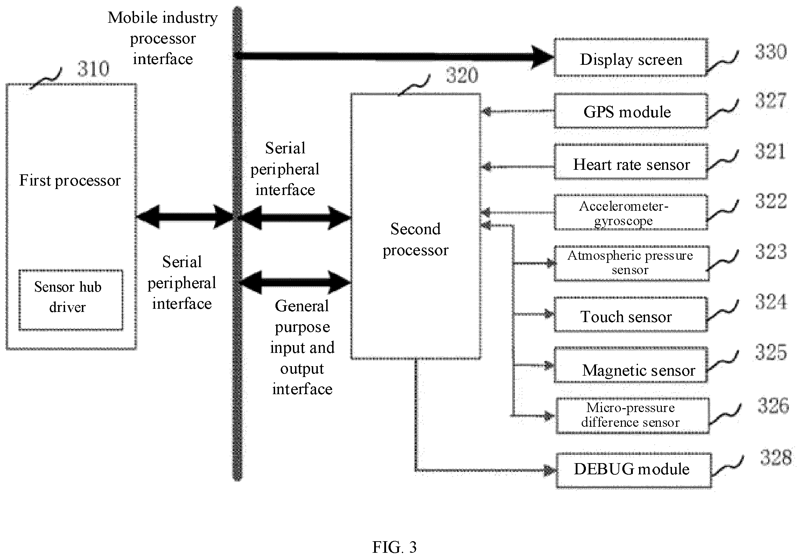

For example, FIG. 3 is a schematic diagram of an internal structure of a wearable device in an embodiment. As illustrated in FIG. 3, in an embodiment, the provided wearable device includes a first processor 310 corresponding to the first system, and a second processor 320 corresponding to the second system. Both the first processor 310 and the second processor 320 are microprocessors, in which the first processor 310 is a core processor. The first processor 310 and the second processor 320 may be configured with corresponding microprocessors based on actual applications. The first processor 310 and the second processor 320 are not specifically limited herein. The system may be, but not limited to, an Android system, a Linux system, a Windows system, an IOS system, an RTOS, and the like. The power consumption of the first system is higher than that of the second system. For example, the first processor 310 may be a Central Process Unit (CPU) processor, and correspondingly, the first system may be an Android system. For example, the second processor 320 may be a Microcontroller Unit (MCU) processor, and correspondingly, the second system may be an RTOS. The CPU may have a main frequency up to 1.2 Gigahertz (GHz), and the MCU may have a main frequency of about 320 Megahertz (MHz). In this case, the power consumption of the first processor is higher than that of the second processor, and the power consumption of the first system is higher than that of the second system.

For example, the wearable device may include one or more of a heart rate sensor 331, an accelerometer-gyroscope 322, an atmospheric pressure sensor 333, a touch sensor 324, a magnetic sensor 325, a micro-pressure difference sensor 326 and other sensors. The second processor 320 may be connected to a sensor included in the wearable device and configured to obtain data collected by the sensor. The second processor 320 may also be connected to: a Global Positioning System (GPS) module 327 configured to obtain positioning data received by the GPS antenna; and a DEBUG module 328 configured to output debugging data of the wearable device.

The first processor 310 and the second processor 320 are connected through a Serial Peripheral Interface (SPI), allowing the first system and the second system to transmit communication data through the SPI bus. A display screen 330 is connected to the first processor 310 and the second processor 320 through a Mobile Industry Processor Interface (MIPI), to display the data output by the first processor 310 or the second processor 320. The first processor 310 includes a sensor hub driver configured to drive data collection and processing of each sensor.

Therefore, the first system runs on the first processor, the second system runs on the second processor. The second processor is connected to the sensor, and the sensor data in the wearable device can be directly obtained through the second system. That is, the sensor data may still be obtained in the second operation mode, which ensures the use of basic functions of the wearable device and reduces the power consumption of the wearable device. In the first operation mode, the first processor is connected to the second processor. Thus, in the first operation mode, the first system and the second system run at the same time, thereby ensuring both the operation of the basic functions of the wearable device and the operation of the extended functions of the wearable device.

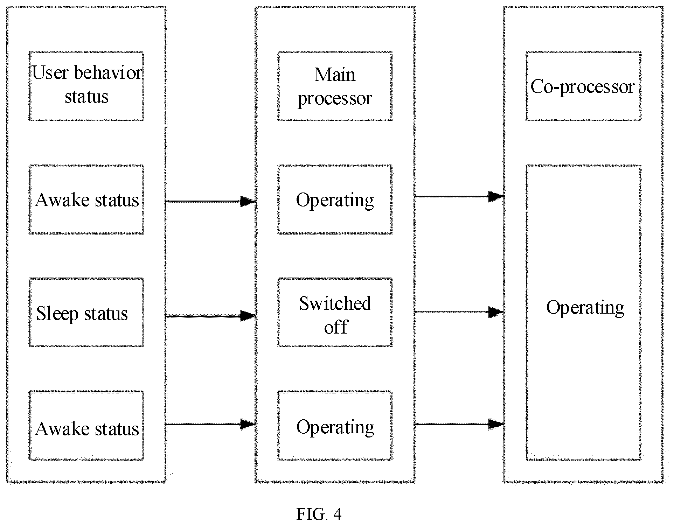

For example, FIG. 4 is a schematic diagram of system switching in an embodiment. When the user behavior status is the awake status, i.e., in the first operation mode, the main processor, i.e., the first processor, is operating, and the co-processor, i.e., the second processor, is also operating. When the user behavior status is the sleep status, i.e., when the user is in the second operation mode, the main processor, i.e., the first processor, does not operate, and the co-processor, i.e., the second processor, is operating. The first processor and the second processor may be connected through an SPIbus. Then, when it is detected that the user behavior status is the sleep status, the wearable device controls the switch between the first processor and the second processor, to disconnect the first processor from the second processor. In this way, the operation mode is switched from the first operation mode to the second operation mode. That is, in the second operation mode, only the second processor is powered on, that is, only the second system is operating.

In the control method for the wearable device in this embodiment, when it is detected that the user behavior status is the sleep status, the switch in the wearable device is controlled to disconnect the first processor from the second processor, and thus the wearable device is switched from the second operation mode to the first operation mode. In this way, the system can be switched without requiring manual operation, and the power consumption of the wearable device can be lowered.

In an embodiment, the control method for the wearable device further includes: switching, in response to detecting that the user behavior status is an awake status, the wearable device from the second operation mode to the first operation mode.

For example, in the second operation mode, the user behavior status can be detected. When the user behavior status is the awake status, the wearable device is switched from the second operation mode to the first operation mode.

The control method for the wearable device in this embodiment can detect the user behavior status no matter in the first operation mode or the second operation mode. When it is detected that the user behavior status is the awake status, the wearable device can be switched from the second operation mode to the first operation mode, without requiring to manually switch the mode. In addition, the wearable device can implement the functions provided by the second processor, as well as functions provided by the first processor, allowing the user to use the wearable device normally.

In an embodiment, said switching, in response to detecting that the user behavior status is the awake status, the wearable device from the second operation mode to the first operation mode includes: controlling, in response to detecting that the user behavior status is the awake status, a switch in the wearable device to connect the first processor corresponding to the first system with the second processor corresponding to the second system, to switch the wearable device from the second operation mode to the first operation mode.

For example, as illustrated in FIG. 4, when the user behavior status is the awake status, i.e., in the first operation mode, the main processor, i.e., the first processor, is operating, and the co-processor, i.e., the second processor, is also operating. Then, when it is detected that the user behavior status is the awake status, the wearable device controls the switch between the first processor and the second processor, to connect the first processor and the second processor, thereby switching the wearable device from the second operation mode to the first operation mode. That is, in the first operation mode, the first processor is connected with the second processor, i.e., both the first system and the second system are running, and both the first processor and the second processor are powered on.

In the control method for the wearable device in this embodiment, when it is detected that the user behavior status is the awake status, the switch in the wearable device is controlled to connect the first processor corresponding to the first system with the second processor corresponding to the second system, to switch the wearable device from the second operation mode to the first operation mode. In this way, the system can be switched without requiring to switch the mode manually. In addition, the wearable device can implement the functions provided by the second processor as well as the functions provided by the first processor, allowing the user to use the wearable device normally.

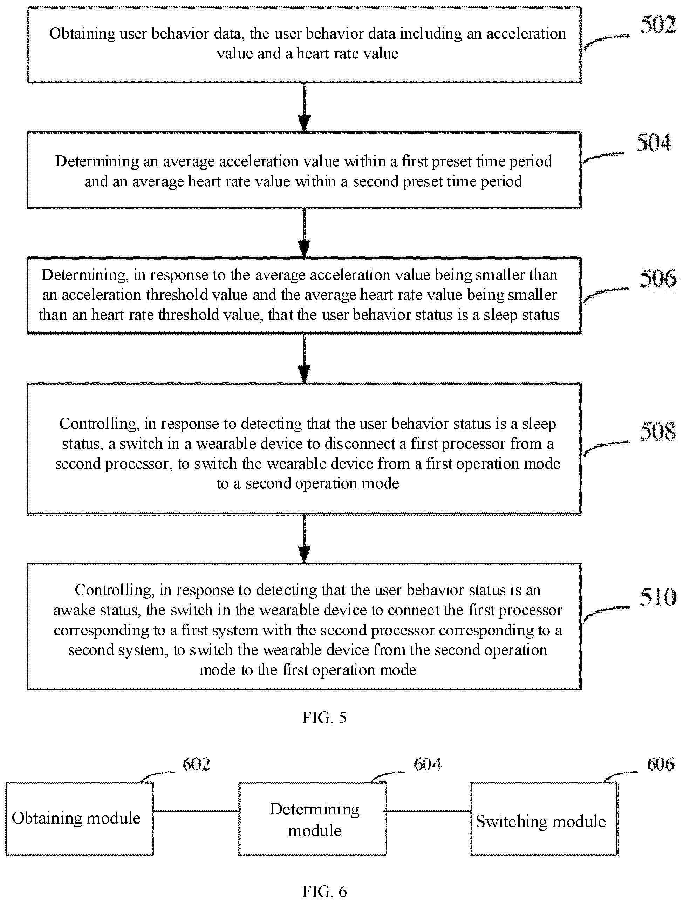

In an embodiment, FIG. 5 is a schematic flowchart of a control method for a wearable device in another embodiment. In the control method for the wearable device, the wearable device has a first operation mode and a second operation mode. The first operation mode is a mode for running a first system and a second system, and the second operation mode is a mode for running only the second system. The first operation mode has a higher power consumption than the second operation mode. The control method includes the following operations.

In operation 502, user behavior data is obtained, and the user behavior data includes an acceleration value and a heart rate value.

In operation 504, an average acceleration value within the first preset time period and an average heart rate value within the second preset time period are determined.

In operation 506, when the average acceleration value is less than an acceleration threshold value and the average heart rate value is less than a heart rate threshold value, it is determined that the user behavior status is a sleep status.

In operation 508, when it is detected that the user behavior status is a sleep status, a switch in the wearable device is controlled to disconnect the first processor from the second processor, to switch the wearable device from the first operation mode to the second operation mode.

In operation 510, when it is detected that the user behavior status is an awake status, the switch in the wearable device is controlled to connect the first processor corresponding to the first system with the second processor corresponding to the second system, to switch the wearable device from the second operation mode to the first operation mode.

In the control method for the wearable device in this embodiment, when switching an operation mode with high power consumption to an operation mode with low power consumption in the sleep status, the system can be switched without requiring the user's manual switching, thereby saving the cost of user operation and lowering the power consumption of the wearable device. When it is detected that the user behavior status is the awake status, the switch in the wearable device is controlled to connect the first processor corresponding to the first system with the second system corresponding to the second system, to switch the wearable device from the second operation mode to the first operation mode, thereby achieving the system switching. In addition, the wearable device can realize the functions provided by the second processor as well as the functions provided by the first processor, allowing the user to use the wearable device normally.

It should be understood that, although the respective operations in the flowcharts of FIG. 1 and FIG. 5 are illustrated in sequence as indicated by arrows, these operations are not necessarily performed in the sequence indicated by the arrows. Unless otherwise specified herein, an execution order of these operations is not strictly limited thereto, and these operations may be performed in other orders. In addition, at least a part of the operations in FIG. 1 and FIG. 5 may include a plurality of sub-operations or a plurality of stages. These sub-operations or stages are not necessarily executed and completed at the same time. However, they may be executed at different time points. These sub-operations or stages are unnecessarily performed in sequence. Instead, they may be performed alternately with other operations or at least a portion of the sub-operations or stages of other operations.

FIG. 6 is a structural block diagram of a control apparatus for a wearable device in an embodiment. As illustrated in FIG. 6, the control apparatus for the wearable device includes an obtaining module 602, a determining module 604, and a switching module 606. The obtaining module 602 is configured to obtain user behavior data. The determining module 604 is configured to determine a user behavior status based on the user behavior data. The switching module 606 is configured to switch, in response to detecting that the user behavior status is a sleep status, the wearable device from the first operation mode to the second operation mode.

The control apparatus for the wearable device in this embodiment can obtain the user behavior data, determine the user behavior status based on the user behavior data, and switch, in response to detecting that the user behavior status is the sleep status, the wearable device from the second operation mode to the first operation mode. That is, when it is necessary to switch from the operation mode with higher power consumption to the operation mode with lower power consumption in the sleep status, the user does not need to manually switch the mode, thereby saving the cost of user operation and lowering the power consumption of the wearable device.

In an embodiment, the user behavior data includes an acceleration value. The determining module 604 is configured to: determine an average acceleration value within a first preset time period; and determine, in response to the average acceleration value being less than an acceleration threshold value, that the user behavior status is the sleep status.

The control apparatus for the wearable device in this embodiment can determine the average acceleration value within the first preset time period, and determine, when the average acceleration value is less than the acceleration threshold value, that the user behavior status is the sleep status. The accidental situation of a single acceleration value may be avoided by the average acceleration value. For example, when the user turns over in sleep or scratches an itch, the instantaneous acceleration value may be high. However, by averaging the values, the accidental situations can be avoided, improving the accuracy of mode switching.

In an embodiment, the user behavior data includes a heart rate value. The determining module 604 is configured to: determine the average heart rate within the second preset time period; and determine, in response to the average heart rate being less than a heart rate threshold value, that the user behavior status is the sleep status.

The control apparatus for the wearable device in this embodiment can determine the average heart rate within the first preset time period, and determine, when the average heart rate is less than the heart rate threshold value, that the user behavior status is the sleep status. The accidental situation of a single heart rate value may be avoided by means of the average heart rate value, which improves the accuracy of mode switching.

In an embodiment, the user behavior data includes an acceleration value and a heart rate value. The determining module 604 is configured to: determine the average acceleration value within the first preset time period and the average heart rate value within the second preset time period; determine, in response to the average acceleration value being less than an acceleration threshold value and the average heart rate value being less than a heart rate threshold value, that the user behavior status is the sleep status.

The control apparatus for the wearable device in this embodiment can determine the average acceleration value within the first preset time period and the average heart rate value within the second preset time period, and determine, when the average acceleration value is less than the acceleration threshold value and the average heart rate value is less than the heart rate threshold value, that the user behavior status is the sleep status. By determining the user behavior state being the sleep state when two conditions are satisfied at the same time, the accuracy of mode switching can be improved.

In an embodiment, the wearable device includes a first processor for running the first system, and a second processor for running the second system. The switching module 606 is configured to control, in response to detecting that the user behavior status is the sleep status, a switch in the wearable device to disconnect the first processor from the second processor to switch the wearable device from the first operation mode to the second operation mode.

The control apparatus for the wearable device in this embodiment can control the switch in the wearable device to disconnect the first processor from the second processor, when it is detected that the user behavior status is the sleep status. Thus, the wearable device can be switched from the second operation mode to the first operation mode, thereby switching the system without requiring the manual operation and lowering the power consumption of the wearable device.

In an embodiment, the switching module 606 is further configured to switch, in response to detecting that the user behavior status is the awake status, the wearable device from the second operation mode to the first operation mode.

The control apparatus for the wearable device in this embodiment can detect the user behavior status no matter in the first operation mode or the second operation mode. When it is detected that the user behavior status is the awake status, the wearable device can be switched from the second operation mode to the first operation mode, without requiring to manually switch the mode. In addition, the wearable device can implement the functions provided by the second processor, as well as functions provided by the first processor, allowing the user to use the wearable device normally.

In an embodiment, the switching module 606 is further configured to control, in response to detecting that the user behavior status is an awake status, a switch in the wearable device to connect the first processor corresponding to the first system with the second processor corresponding to the second system, to switch the wearable device from the second operation mode to the first operation mode.

When it is detected that the user behavior status is the awake status, the control apparatus for the wearable device in this embodiment can control the switch in the wearable device to connect the first processor corresponding to the first system with the second processor corresponding to the second system. Thus, the wearable device can be switched from the second operation mode to the first operation mode, and thus the system can be switched without requiring to manually switch the mode. The wearable device can implement the functions provided by the second processor as well as the functions provided by the first processor, allowing that the user to use the wearable device normally.

The division of the respective modules in the above-mentioned control apparatus for the wearable device is only used for illustration. In other embodiments, the control apparatus for the wearable device may be divided into different modules as needed, so as to complete all or part of the functions of the control apparatus for the wearable device.

For the specific limitations of the control apparatus for the wearable device, reference may be made to the limitations of the control method for the wearable device as described above, which will not be repeated herein. Each module in the above control apparatus for the wearable device may be implemented in whole or in part by software, hardware and combinations thereof. The above modules may be embedded in or independent of a processor in a computer device in the form of hardware, or they may be stored in a memory in a computer device in the form of software to allow the processor to call and execute the operations corresponding to the above modules.

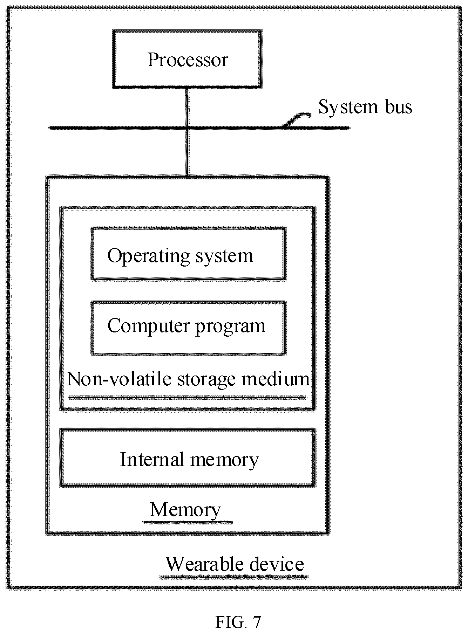

FIG. 7 is a schematic diagram of an internal structure of a wearable device in an embodiment. As illustrated in FIG. 7, the wearable device includes a processor and a memory that are connected to each other through a system bus. The processor is configured to provide computing and control capabilities, for supporting the operations of the entire electronic device. The memory may include a non-volatile storage medium and an internal memory. The nonvolatile storage medium stores an operating system and a computer program. The computer program may be executed by the processor to implement a control method for a wearable device provided by the following embodiments. The internal memory provides a cached execution environment for operating system computer programs in the non-volatile storage medium. The wearable device may be a terminal device such as a watch and a wristband.

The respective modules in the control apparatus for the wearable device provided in the embodiments of the present disclosure may be embodied in the form of a computer program. The computer program may be run on a terminal or server. The program module constituted by the computer program may be stored on a memory of an electronic device. When the computer program is executed by the processor, the operations of the methods described in the embodiments of the present disclosure are implemented.

Embodiments of the present disclosure further provide a computer-readable storage medium. For one or more non-volatile computer-readable storage media containing computer-executable instructions, the computer-executable instructions, when being executed by one or more processors, cause the one or more processors to perform the operations of the control method for the wearable device.

A computer program product containing instructions, when running on a computer, causes the computer to execute the control method for the wearable device.

Any reference to a memory, storage, database, or other medium as used herein may include a non-volatile and/or volatile memory. The non-volatile memory may include Read Only Memory (ROM), Programmable ROM (PROM), Electrically Programmable ROM (EPROM), Electrically Erasable Programmable ROM (EEPROM), or flash memory. The volatile memory may include Random Access Memory (RAM), which acts as external cache memory. By way of illustration but not limitation, RAM is available in various forms such as Static RAM (SRAM), Dynamic RAM (DRAM), Synchronous DRAM (SDRAM), Double Data Rate SDRAM (DDR SDRAM), Enhanced SDRAM (ESDRAM), Synchronous Link (Synchlink) DRAM (SLDRAM), Memory Bus (Rambus) Direct RAM (RDRAM), Direct Memory Bus Dynamic RAM (DRDRAM), and Memory Bus Dynamic RAM (RDRAM).

The above embodiments only represent several embodiments of the present disclosure, and the description thereof is relatively specific and detailed. However, they should not be construed as limitations on the scope of the patent of the present disclosure. It should be noted that those skilled in the art, without departing from the concept of the present disclosure, can make several modifications and improvements, which shall fall within the protection scope of the present disclosure. The protection scope of the present disclosure shall be defined by the appended claims.

## Claims

1. A control method for a wearable device, the wearable device having a first operation mode and a second operation mode, the first operation mode being a mode for running a first system and a second system, the second operation mode being a mode for running only the second system, and the first operation mode having a higher power consumption than the second operation mode,
the control method comprising:
obtaining user behavior data;
determining a user behavior status based on the user behavior data; and
switching, in response to detecting that the user behavior status is a sleep status, the wearable device from the first operation mode to the second operation mode.

2. The control method according to claim 1, wherein the user behavior data comprises an acceleration value; and
wherein said determining the user behavior status based on the user behavior data comprises:
determining an average acceleration value within a first preset time period; and
determining, in response to the average acceleration value being less than an acceleration threshold value, that the user behavior status is the sleep status.

3. The control method according to claim 1, wherein the user behavior data comprises a heart rate value; and
wherein said determining the user behavior status based on the user behavior data comprises:
determining an average heart rate within a second preset time period; and
determining, in response to the average heart rate being less than a heart rate threshold value, that the user behavior status is the sleep status.

4. The control method according to claim 1, wherein the user behavior data comprises an acceleration value and a heart rate value; and
wherein said determining the user behavior status based on the user behavior data comprises:
determining an average acceleration value within a first preset time period and an average heart rate value within a second preset time period; and
determining, in response to the average acceleration value being less than an acceleration threshold value and the average heart rate value being less than a heart rate threshold value, that the user behavior status is the sleep status.

5. The control method according to claim 1, wherein the wearable device comprises:
a first processor for running the first system; and
a second processor for running the second system,
wherein said switching, in response to detecting that the user behavior status is the sleep status, the wearable device from the first operation mode to the second operation mode comprises:
controlling, in response to detecting that the user behavior status is the sleep status, a switch in the wearable device to disconnect the first processor from the second processor, to switch the wearable device from the first operation mode to the second operation mode.

6. The control method according to any one of claims 1 to 5, further comprising:
switching, in response to detecting that the user behavior status is an awake status, the wearable device from the second operation mode to the first operation mode.

7. The control method according to claim 6, wherein said switching, in response to detecting that the user behavior status is the awake status, the wearable device from the second operation mode to the first operation mode comprises:
controlling, in response to detecting that the user behavior status is the awake status, a switch in the wearable device to connect the first processor corresponding to the first system with the second processor corresponding to the second system, to switch the wearable device from the second operation mode to the first operation mode.

8. A control apparatus for a wearable device, wherein the wearable device having a first operation mode and a second operation mode, the first operation mode comprising a mode for running a first system and a second system, the second operation mode being a mode for running only the second system, and the first operation mode having a higher power consumption than the second operation mode;
the control apparatus comprising:
an obtaining module configured to obtain user behavior data;
a determining module configured to determine a user behavior status based on the user behavior data; and
a switching module configured to switch, in response to detecting that the user behavior status is a sleep status, the wearable device from the first operation mode to the second operation mode.

9. The control apparatus according to claim 8, wherein the user behavior data comprises an acceleration value; and wherein the determining module is configured to:
determine an average acceleration value within a first preset time period; and
determine, in response to the average acceleration value being less than an acceleration threshold value, that the user behavior status is the sleep status.

10. The control apparatus according to claim 8, wherein the user behavior data comprises a heart rate value; and wherein the determining module is configured to:
determine an average heart rate within a second preset time period; and
determine, in response to the average heart rate being less than a heart rate threshold value, that the user behavior status is the sleep status.

11. The control apparatus according to claim 8, wherein the user behavior data comprises an acceleration value and a heart rate value; and wherein the determining module is configured to:
determine an average acceleration value within a first preset time period and an average heart rate value within a second preset time period; and
determine, in response to the average acceleration value being less than an acceleration threshold value and the average heart rate value being less than a heart rate threshold value, that the user behavior status is the sleep status.

12. The control apparatus according to claim 8, wherein the wearable device comprises:
a first processor for running the first system; and
a second processor for running the second system,
wherein the switching module is configured to:
control, in response to detecting that the user behavior status is the sleep status, a switch in the wearable device to disconnect the first processor from the second processor, to switch the wearable device from the first operation mode to the second operation mode.

13. The control apparatus according to any one of claims 8 to 12, wherein the switching module is further configured to:
switch, in response to detecting that the user behavior status is an awake status, the wearable device from the second operation mode to the first operation mode.

14. The control apparatus according to claim 13, wherein the switching module is further configured to:
control, in response to detecting that the user behavior status is the awake status, a switch in the wearable device to connect the first processor corresponding to the first system with the second processor corresponding to the second system, to switch the wearable device from the second operation mode to the first operation mode.

15. An electronic device, comprising
a memory having a computer program stored thereon; and
a processor,
wherein the processor is configured to, when executing the computer program, implement steps of the control method for the wearable device according to any one of claims 1 to 7.

16. A computer-readable storage medium, having a computer program stored thereon, wherein the computer program, when being executed by a processor, implements steps of the control method according to any one of claims 1 to 7.
